# EUROPEAN PATENT SPECIFICATION

(11) **EP 3 148 547 B1**
(45) Date of publication and mention of the grant of the patent: **03.01.2024**
(21) Application number: 15802818.3
(22) Date of filing: 02.06.2015
(51) Int. Cl.: A61K 31/517, A61K 31/5377, A61K 31/5415, A61P 35/00, A61P 35/04

(54) **METHOD FOR TREATING DRUG RESISTANT CANCER**
VERFAHREN ZUR BEHANDLUNG VON ARZNEIMITTELRESISTENTEM KREBS
PROCÉDÉ DE TRAITEMENT D'UN CANCER PHARMACORÉSISTANT

(30) Priority: 02.06.2014 US 201462006630 P
(43) Date of publication of application: 05.04.2017
(73) Proprietor: National Yang-Ming University, Taipei 112 (TW)
(72) Inventor: CHANG, Peter Mu-Hsin, Taipei 112 (TW); CHEN, Kuan-Yu, Taipei 112 (TW); WU, Chun-Hung, Taipei 112 (TW); CHENG, Tai-Shan, Taipei 112 (TW); YU, Cheng-Hao, Taipei 112 (TW)
(74) Representative: Straus, Alexander
(86) International application number: PCT/CN2015/000380
(87) International publication number: WO 2015/184794

(56) References cited:
- WO-A1-2012/158657
- WO-A1-2013/070688
- WO-A2-2012/149186
- HARUHIRO SAITO ET AL: "Effectiveness of Erlotinib in Advanced Non-Small Cell Lung Cancer in Cases of Gefitinib Resistance after Treatment of More Than 6 Months", ONKOLOGIE, vol. 35, no. 1-2, 1 January 2012 (2012-01-01), pages 18-22, XP055427032, DE ISSN: 0378-584X, DOI: 10.1159/000335736
- RASCHKO J W ET AL: "A phase I study of carboplatin and etoposide administered in conjunction with dipyridamole, prochlorperazine and cyclosporine A", CANCER CHEMOTHERAPY AND PHARMACOLOGY, SPRINGER VERLAG, BERLIN, vol. 46, no. 5, 1 January 2000 (2000-01-01), pages 403-410, XP009501613, ISSN: 0344-5704
- KRISHAN A ET AL: "Synergistic effect of prochlorperazine and dipyridamole on the cellular retention and cytotoxicity of doxorubicin", CLINICAL CANCER RESEARCH, THE AMERICAN ASSOCIATION FOR CANCER RESEARCH, US, vol. 6, no. 4, 31 March 2000 (2000-03-31), pages 1508-1517, XP009501614, ISSN: 1078-0432
- SRIDHAR K S ET AL: "Phase I and pharmacokinetics studies of prochlorperazine 2-h i.v. infusion as a doxorubicin-efflux blocker", CANCER CHEMOTHERAPY AND PHARMACOLOGY, SPRINGER VERLAG, BERLIN, vol. 34, no. 5, 1 January 1994 (1994-01-01), pages 377-384, XP009501752, ISSN: 0344-5704

## Description

### CROSS REFERENCE

This application claims priority to U.S. Provisional Patent Appl. No. 62/006,630, filed on June 2, 2014.

### FIELD OF THE INVENTION

The present invention relates to a method for treating a gefitinib resistant cancer, in particular a lung cancer, with prochlorperazine in combination with gefitinib.

Any references in the description to methods of treatment refer to the compounds, pharmaceutical compositions and medicaments of the present invention for use in a method for treatment of the human (or animal) body by therapy.

### BACKGROUND OF THE INVENTION

Chemotherapy, particularly with a combination of anti-cancer agents, is the treatment of choice for delocalized tumors that are untreatable by surgery or radiation. However, some patients relapse after even a short period of time, and do not respond to a second course of chemotherapy.

Most malignant tumors show some sensitivity to cytotoxic drugs. Accordingly, treatment with these drugs generally provides remission and shrinkage of the tumor that may last for weeks to months. Nonetheless, in many cases the tumors regrow, and this regrowth is resistant to further cytotoxic treatments.

Theoretically it should be possible to solve this problem by administering a combination of drugs that act differently. This method of treatment is based upon the extremely small probability that two or more different drug resistances would arise spontaneously in the same cell. Combination chemotherapy appeared to obviate the problem of drug-resistant tumor cells.

Research was then directed to protocols for administering anticancer drugs in combinations. Newly developed drugs and combination chemotherapy several decades ago produced high cure rates for some childhood leukemia and for Hodgkin's disease. However, the major killers, such as lung cancer, breast cancer, and cancers of the gastrointestinal tract, remained resistant to chemotherapy. The failures of combination chemotherapy were not understandable. Many theories were proposed to explain the observations, but few of these theories could be adequately tested. CN1303858 (A) discloses A tetrahydroxytoluylene glucoside type new compound of a formular as disclosed herein below for the use in the treatment of cardiovascular disorders. Zhang et al. (Zhang Wei, et al., Clinical and Experimantal Pharmacology and Physiology, vol. 35(3), p. 310-316, March 3, 2008; and further Zhang et al., Journal of China Pharmaceutical University, Nanjing, vol. 38(3), p. 261-264, January 1, 2007) show the use of the substance as disclosed herein below in the treatment of atherosclerosis. Raschko et al. (Raschko, J., et al. Cancer Chemother Pharmacol vol. 46, p. 403-410 (2000) inter alia found that in vitro drug resistance-reversing levels of prochlorperazine can be achieved in vivo, and found a significant effect on the pharmacokinetics of etoposide. Krishan et al. (Krishan A. et al., Clin Cancer Res, 2000 Apr; vol. 6(4):p. 1508-17) found that an incubation of drug-resistant human tumor cells with a combination of prochlorperazine and dipyridamole has synergistic effects on the cellular retention and cytotoxicity of doxorubicin. Sridhar et al. (Sridhar KS et al., Cancer Chemother Pharmacol. 1994; vol. 34(5): p. 377-84.) found that prochlorperazine levels high enough to block doxorubicin efflux in vitro could be achieved and sustained in vivo. WO2012149186 A2 discloses novel bisaminoquinoline compounds and pharmaceutical compositions comprising these compounds for inhibiting autophagy in biological systems in order to treat cancer in patients in combination inter alia with prochlorperazine. WO2013070688 A1 discloses compositions for co-administration of anti-cancer agents inter alia comprising prochlorperazine to treat metastatic cancer where cancer cells exhibit overexpression (heightened expression) of cell surface urokinase-type plasminogen activator receptor (urokinase receptor) compared to normal (non-cancerous) cells.

Although significant strides have been made in the development of pharmaceutical compositions for treating cancer, new methods of treating drug resistant cancer are required.

### SUMMARY OF THE INVENTION

It was unexpectedly found that prochlorperazine in combination with a particular chemotherapeutic drug exhibits a synergistic effect in reducing the size and number of the cancer cells, and inhibiting the growth of cancer cells with drug resistance properties.

The disclosure relates to a method, not forming part of the invention, for treating a subject with a lung cancer resistant to a chemotherapeutic drug or for preventing lung cancer metastasis. The method comprises administering to said subject a therapeutically effective amount of prochlorperazine or a pharmaceutically acceptable salt thereof, in combination the chemotherapeutic drug. The scope of the invention shall be defined by the appended claims.

In one aspect, the invention provides a pharmaceutical composition for treating a subject with a lung cancer resistant to gefitinib, comprising prochlorperazine or a pharmaceutically acceptable salt thereof, in combination with gefinitib.

In one yet aspect, the disclosure provides a use of prochlorperazine or a pharmaceutically acceptable salt thereof, in combination with gefitinib, for manufacturing a medicament for treating a subject with a lung cancer resistant to gefitinib.

In one further yet aspect, the disclosure provides a use of prochlorperazine or a pharmaceutically acceptable salt thereof, for manufacturing a medicament for preventing lung cancer metastasis in combination with erlotinib.

In one embodiment, the invention provides a pharmaceutical composition for use in preventing lung cancer metastasis; which comprises prochlorperazine or pharmaceutical acceptable salt thereof in combination with erlotinib.

In one further aspect, the metastasis is prevented in lung squamous cell carcinoma.

### BRIEF DESCRIPTION OF THE DRAWINGS

The foregoing summary, as well as the following detailed description of the invention, will be better understood when read in conjunction with the appended drawing. In the drawings:
**Figure 1** showed that prochlorperazine induced apoptosis and synergistically enhanced cytotoxicity in combination of gemcitabine in CL152 spheres; wherein
**Figure 1(A)** showed that CL152 spheres were collected and analyzed by flow cytometry after treatment with prochlorperazine for 48 hours. Prochlorperazine led to a dose-dependent increase in apoptosis.
**Figure 1(B)** showed that prochlorperazine had synergistic effects with gemcitabine in CL152 spheres (not according to invention).
**Figure 1(C)** showed that prochlorperazine inhibited cell migration after prochlorperazine treatment.
**Figure 1(D)** showed that prochlorperazine increased the number of p-galactosidase positive cells and induced senescence at A549 cells treated with prochlorperazine for 24 hours. A549 cells were treated with 50 µM resveratrol as a senescent positive control.
**Figure 2** showed that prochlorperazine reduced the percentage of cancer stem-like cells and enhanced chemotherapeutic agents and gefitinib induced cytotoxicity; wherein
**Figure 2(A)** showed that prochlorperazine decreased cell survival of CL141 cancer stem-like sphere cells and enhanced anti-cancer activity of pemetrexed;
**Figure 2(B)** showed that prochlorperazine decreased cell survival of CL97 cancer stem-like sphere cells and enhanced anti-cancer activity of gefitinib;
**Figure 2(C)** showed that prochlorperazine inhibited sphere formation activity of HCC827 cells and enhanced anti-cancer activity of cisplatin (not according to invention);
**Figure 2(D)** showed that prochlorperazine inhibited sphere formation activity of H1299 cells and enhanced anti-cancer activity of cisplatin (not according to invention);
**Figure 3** provides that the in vivo monitoring of prochlorperazine-mediated antitumor effects either alone and in combination of a standard chemotherapeutic agent; wherein:
   **Figure 3(A)** showed that 5 × 105 H441 bulk tumor cells were subcutaneously injected into the right flank ofNOD/SCID mice which were subsequently divided into vehicle (control) and prochlorperazine (5 mg/kg/day, 5 times a week). Tumor burden was measured using a caliper and fold change in tumor size was plotted over time; our initial results demonstrated that at this concentration, prochlorperazine alone suppressed (or delayed) tumorigenesis in vivo. In subsequent experiments, we used prochlorperazine in combination of a standard chemotherapeutic agent for tumor suppressive effects.
   **Figure 3(B)** indicated that the combination of pemetrexed (1 mg/kg, 5 times a week) and prochlorperazine (1mg/kg, 5 times a week) provided the most significant tumor suppressive effect as compared to control, pemetrexed alone (1mg/kg, 5 times a week) and the combination of pemetrexed (1 mg/kg, 5 times a week) and cisplatin (1mg/kg, twice a week) (not according to invention).
   **Figure 3(C)** shows the results of the experiments on adenocarcinoma tumor model, wherein the standard chemotherapeutic regimen is the combination of pemetrexed (50 mg/kg) and cisplatin (3 mg/kg); and the results show that the addition of prochlorperazine (5 mg/kg) with the standard regimen yielded the least tumor burden followed by prochlorperazine alone (5 mg/kg), the combination of pemetrexed (50 mg/kg) and cisplatin (3 mg/kg) and the vehicle control (not according to invention).
   **Figure 3(D)** shows the results of the experiments on squamous model with a standard treatment with the combination of gemcitabine and cisplatin; indicating that the addition of prochlorperazine (5 mg/kg) to the standard treatment (the combination of 60 mg/kg gemcitabine and 3 mg/kg cisplatin) provided the most tumor suppressive effect followed by prochlorperazine alone (5 mg/kg), the combination of gemcitabine and cisplatin, and the vehicle control.
   **Figure 3 (E)** demonstrates the results of the treatment with gefitinib in addition of prochlorperazine on gefitinib-resistant NSCLC model; wherein the combination of gefitinib (100mg/kg) and prochlorperazine (5mg/kg) showed the highest degree of tumor inhibition followed by prochlorperazine alone (5mg/kg); gefitinib alone (100mg/kg) and the vehicle control groups showed similar tumor burden.

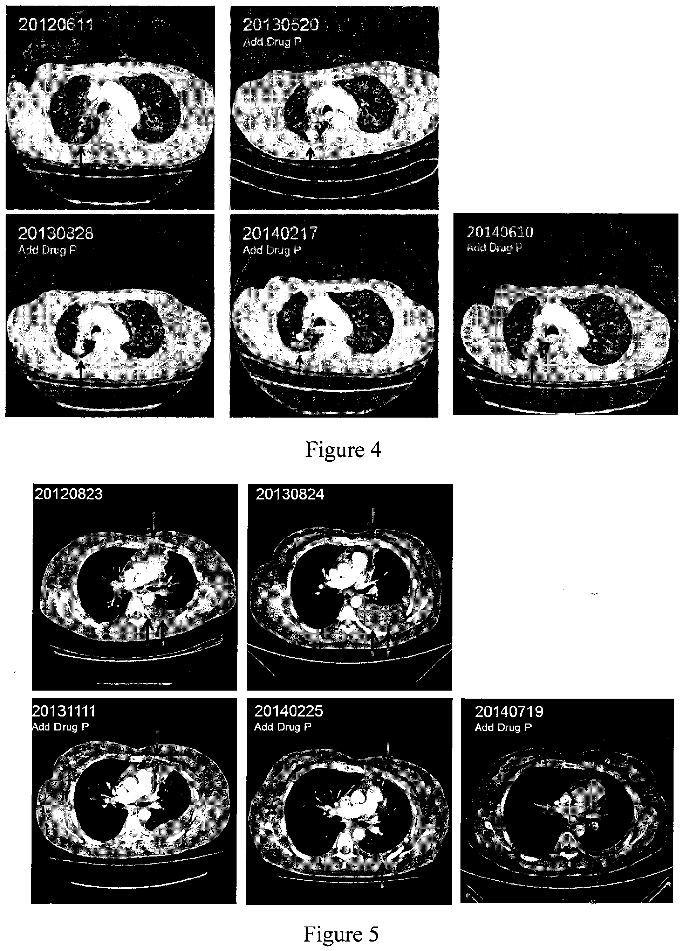

   **Figure 4** provides some images showing the results of a case study using prochlorperazine for lung squamous cell carcinoma patient wherein the patient is a 81 y/o male with lung squamous cell carcinoma, right lower lung with right upper lung metastasis. The patient accepted radiation over right lower lung primary lesion due to hemoptysis; and then received Tarceva^{™}(containing erlotinib as active ingredient) since May 19,2010. The condition kept stable and first CT showed a 0.5 cm right upper lung metastasis. This lesion progressed to 2 cm on May 20, 2013 with worsening cough and dyspnea. Due to old age and poor general condition, he decided to continue Tarceva^{™} with only adding prochlorperazine. After taking prochlorperazine, the cough and dyspnea improved and followed CT after 3 months showed stable disease. The regimen was continued until last followed-up CT on 2014/06/10 showed the tumor progression. The patient died at the end of 2014.
   **Figure 5** provides some images showing the results of a case study using prochlorperazine for lung adenocarcinoma patient. Lung adenocarcinoma patient (50 y/o female, LUL) carried with EGFR-L858R mutation was first treated with gefitinib since 2012 (with malignant pleural effusion) and pemetrexed since August 24, 2013. The patient was then took prochlorperazine along with pemetrexed on November 11, 2013. The regimen was continued and last followed-up CT on July 19, 2014, showing that the tumor and pleural effusion were reduced significantly. The patient still survives now.

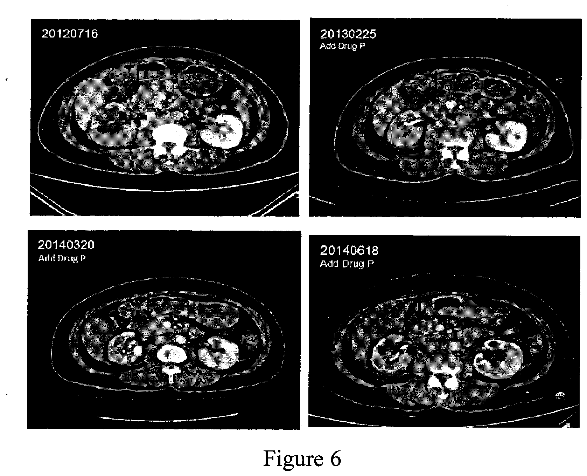

   **Figure 6** provides some images showing the results of a case study using prochlorperazine for signet ring cell carcinoma patient. Signet ring cell carcinoma patient (58 y/o female), with multiple intra-abdominal metastasis, which has average median survival 7-8 months, were treated with several kinds of chemotherapy (cisplatin, 5-fluorouracil, irinotecan, 10 paclitaxel, gemcitabine, avastin, TS-1, oxaliplatin, paclitaxel/5-fluorouracil and avastin/gemcitabine/TS-1) since July 16,2012. Patient took prochlorperazine on February 25, 2013. The regimen was continued and last followed-up CT on June 18, 2014, showing that the tumor was stable. The patient died at the end of 20 14 (not according to invention).

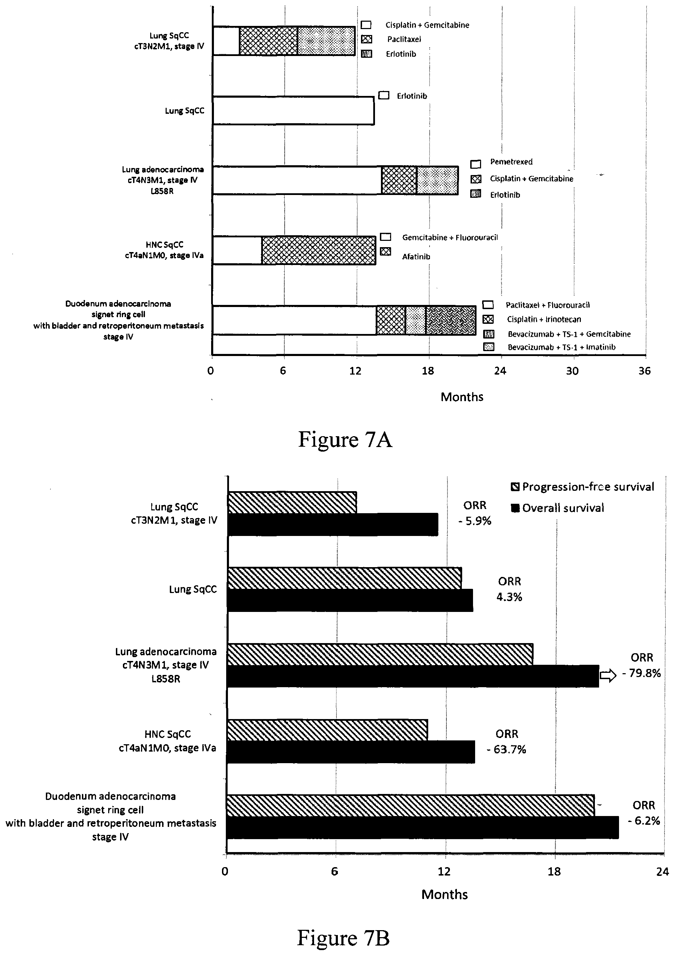

   **Figure 7A and 7B** showed the clinical course and the addition of prochlorperazine as maintenance therapy. All of the caseswere treated according to clinical guideline. Life expectancy of all patients was less than 3 months. Prochlorperazine was added with salvage therapy to prolong its effective duration from drug resistance. The aim of such maintenance therapy is to help control the disease without progression, allowing patients to live longer. The objective response rate (ORR), progression-free survival (PFS), and overall survival (OS) after prochlorperazine treatment showed that the use of maintenance therapy indeed effectively kept disease status stable and prolonged survivals. Median ORR was -6.2% (-79.8% - 4.3%). Median PFS and OS after prochlorperazine treatment was 12.8(7.0-20.1) months and 13.5(7.4-21.4) months, respectively.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

As used herein, the term "prochlorperazine" refers to a dopamine (D2) receptor antagonist that is used for the antiemetic treatment of nausea and vertigo. Prochlorperazine has the structure of

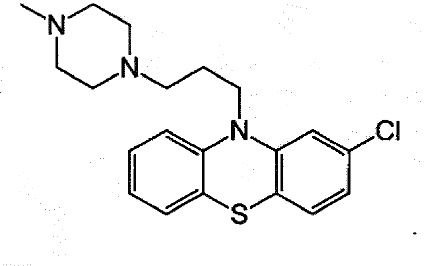

As used herein the term "pharmaceutically acceptable salt" refers to any pharmaceutically acceptable salt of prochlorperazine. The pharmaceutically acceptable salts include ammonium salts, alkali metal salts such as potassium and sodium (including mono, di- and tri-sodium) salts (which are preferred), alkaline earth metal salts such as calcium and magnesium salts, salts with organic bases such as dicyclohexylamine salts, N-methyl-D-glucamine, and salts with amino acids such as arginine, lysine, and so forth.

As used herein, the term "subject" refers to any warm-blooded species such as humans and animals. The subject, such as a human, to be treated according to the present invention may in fact be any subject of the human population, male or female, which may be divided into children, adults, or elderly. Anyone of these patient groups relates to an embodiment of the invention.

The disclosure relates to a method, not forming part of the invention, for treating a subject with a cancer resistant to a chemotherapeutic drug. The method comprises administering to said subject a therapeutically effective amount of prochlorperazine or a pharmaceutically acceptable salt thereof, in combination of the chemotherapeutic drug.

In some examples of the present disclosure, the combination of prochlorperazine and a chemotherapeutic drug exhibits a synergistic effect in reducing the size and number of the cancer cells. In other examples of the present disclosure, the combination of prochlorperazine and a chemotherapeutic drug exhibits a synergistic effect in inhibiting the growth of cancer cells.

As used herein, the term "chemotherapeutic drug" may refer to gefitinib and erlotinib.

It was found that some metabolites have the same cytotoxic activities as prochlorperazine, as shown in Table 2, however, these metabolites do not form part of the invention.

It was also found in the examples of the invention that some analogs have the same: clonogenic activities as prochlorperazine, as shown in Table 3, however, these analogs do not form part of the invention.

As used herein, the term "therapeutically effective amount" refers to an amount sufficient for providing an effect in treatment for a cancer, which is depending on the mode of administration and the condition to be treated, including age, body weight, symptom, therapeutic effect, administration route and treatment time.

In the present invention, various lung cancers can be treated with the pharmaceutical composition. The cancer is selected from the group consisting of lung cancers, including adenocarcinoma, squamous cell carcinoma, and large cell carcinoma.

It is found in the example of the disclosure that prochlorperazine exhibited cytotoxicity in various types of cancers, including adenocarcinoma, squamous cell carcinoma, large cell carcinoma, liver cancer, colorectal adenocarcinoma, brain cancer, breast cancer, pancreatic cancer, and myeloma, see Table 1, however, the composition of the invention is only for use in treating of lung cancer types.

The present disclosure relates to a method, not forming part of the invention, for treating a subject with a lung cancer resistant to a chemotherapeutic drug. The method comprises administering to said subject a therapeutically effective amount of prochlorperazine or a pharmaceutically acceptable salt thereof, in combination of the chemotherapeutic drug. The lung cancer may be a non-small cell lung carcinoma (NSCLC).

On the other hand, the present disclosure relates to a method, not forming part of the invention, for preventing lung cancer metastasis. The method comprises administering to a subject in need thereof a therapeutically effective amount of prochlorperazine or a pharmaceutically acceptable salt thereof, in combination of a chemotherapeutic drug. The lung cancer may be a lung squamous cell carcinoma.

In one example of the invention, prochlorperazinein in combination with erlotinib provided an effect in prevention of metastasis of a lung cancer, such as lung squamous cell carcinoma.

In addition, the disclosure provides a use of prochlorperazine or a pharmaceutically acceptable salt thereof, in combination with gefitinib, for manufacturing a medicament for treating a subject with a lung cancer resistant to a chemotherapeutic drug. The disclosure also provides a use of prochlorperazine or a pharmaceutically acceptable salt thereof, in combination with erlotinib, for manufacturing a medicament for preventing cancer metastasis.

The pharmaceutical composition or formulation, may be formulated to be administered in any route that is appropriate, including but not limited to oral or parenteral administration. The composition or formulation of the invention may be administered through oral route, which may be in a solid or liquid form. The solid compositions or formulations include tablets, pills, capsules, dispersible powders, granules, and the like. The oral compositions also include gargles which are to be stuck to oral cavity and sublingual tablets. The capsules include hard capsules and soft capsules. In such solid compositions or formulations for oral use, one or more of the active compound(s) may be admixed solely or with diluents, binders, disintegrators, lubricants, stabilizers, solubilizers, and then formulated into a preparation in a conventional manner. When necessary, such preparations may be coated with a coating agent, or they may be coated with two or more coating layers. On the other hand, the liquid compositions for oral administration include pharmaceutically acceptable aqueous solutions, suspensions, emulsions, syrups, elixirs, and the like. In such compositions, one or more of the active compound(s) may be dissolved, suspended or emulsified in a commonly used diluent (such as purified water, ethanol or a mixture thereof, etc.). Besides such diluents, said compositions may also contain wetting agents, suspending agents, emulsifiers, sweetening agents, flavoring agents, perfumes, preservatives and buffers and the like.

The pharmaceutical compositions for parenteral administration include solutions, suspensions, emulsions, and solid injectable compositions that are dissolved or suspended in a solvent immediately before use. The injections may be prepared by dissolving, suspending or emulsifying one or more of the active ingredients in a diluent. Examples of said diluents are distilled water for injection, physiological saline, vegetable oil, alcohol, and a combination thereof. Further, the injections may contain stabilizers, solubilizers, suspending agents, emulsifiers, soothing agents, buffers, preservatives, etc. The injections, are sterilized in the final formulation step or prepared by sterile procedure. The pharmaceutical composition of the invention may also be formulated into a sterile solid preparation, for example, by freeze-drying, and may be used after sterilized or dissolved in sterile injectable water or other sterile diluent(s) immediately before use.

The examples below are to be construed as merely illustrative. Without further elaboration, it is believed that one skilled in the art can, based on the description herein, utilize the present invention to its fullest extent.

### Examples

### I. Cell culture and Chemicals

A549, CLl41 and H441 are EGFR-wild type adenocarcinoma cell lines, HCC827 has EGFR-exon 19 deletion, and CL97 is an EGFR T790M and G719A mutations cell line. CL152, H2170 and H226 are squamous cell carcinoma cell lines and H1299 is a non-small cell lung carcinoma cell line. A549-0N cell line is A549 cell overexpression Oct4 and Nanog which we regarded it as cancer stem cell-like cell line (22). All cell lines were maintained in RPMI medium and supplemented with 10% fetal bovine serum (FBS, Invitrogen), 2 mM L-glutamine, 100 U/mL penicillin, and 100 ug/ml. streptomycin. For cell culture experiment, 10mM prochlorperazine stock solution, was dissolved in dimethyl sulfoxide (DMSO; Sigma).Prochlorperazine, Cisplatin, Gemcitabine were purchased from Sigma.

### II. Cytotoxicity and sulforhodamine B assay

Cells were plated in 96-well plates at a density of 2000 cells per well in triplicate. The cells were treated on the third day (to ensure proper plating efficiency and vitality) to indicated agents for 48hrs. Cells were treated with different concentrations of prochlorperazine, cisplatin, gemcitabine, or a combination of, for example, prochlorperazine and gemcitabine. Cytotoxicity was assessed using the sulforhodamine B (SRB) assay (23). Briefly, the medium was discarded, and the adherent cells were fixed by 100 /-11 of cold 10% trichloroacetic acid (w/v) in each well for 1 h at 4°C. After fixation, cells were stained with 100 µl/well of 0.4% (w/v, in 1% acetic acid) SRB solution for 30 min at room temperature, and then washed 5 times with 1% 2:' acetic acid. After air-drying, 100 /-11 of 10mM Tris base was added to each well and the absorbance was read at 530 nm. Cytotoxicity is expressed as the percent of cells in drug treated wells relative to number of cells in the solvent only control (set to 100%). Each experiment was performed independently at least 2 times in triplicate and cytotoxicity is given as means ±SD.

### III. Clonogenic assay

Tested cells were seeded respectively in 6 well plates with 104 cells per well for 14 days. Prochlorperazine and other tested drugs were added 24 hours after seeding of the cells. The medium and prochlorperazine were changed every 4 days. After the treatments, cells were washed with PBS, and the colonies were fixed with fix solution (methanol: acetic acid = 3:1) and stained with 0.5% crystal violet in methanol. After removing the crystal violet carefully and rinse with tap water, the colonies were counted manually. Each experiment was performed independently at least 2 times in triplicate and cytotoxicities are given as means ±SD.

### IV. Tumor spheroid assay

In brief, single cells were plated in 6-well ultralow attachment plates (Corning Inc.) at a density of 2,000 cells/mL in tumor spheroid culture medium, DMEM/FI2 supplemented with 1% N2 Supplement (Invitrogen), 10 ng/mL basic fibroblast growth factor (Sigma-Aldrich), 10 ng/mL epidermal growth factor (Invitrogen) with I% penicillin/streptomycin (Invitrogen) at 37 "C in a humidified atmosphere of 95% air and 5% C02. Cells were cultured twice per week. When passaged, tumor spheres were harvested. Spheroids were dissociated with TrypLETM (Invitrogen). Spheroids cell counting using the Trypan Blue Exclusion method.

### V. Side population analysis and purification using flow cytometry

Single-cell suspensions of cells were detached from dishes with TrypsinEDTA (Invitrogen) and suspended at I x 106 cells/mL in Hank's balanced salt solution (HBSS) supplemented with 3% fetal calf serum and 10mM Hepes. These cells were then incubated at 37°C for 90 minutes with 20 µg/ml, Hoechst 33342 (Sigma Chemical, St. Louis, MO). The ABC transporter inhibitor verapamil (Sigma) was added at a final concentration of 50 ~M to confirm the gating area on flow cytometry. After 90 minutes incubation with indicated drugs, the cells were centrifuged immediately for 5 minutes at 300 g and 4 °C and resuspended in ice-cold HBSS. The cells were kept on ice to inhibit efflux of the Hoechst dye, and I µg/ml. propidium iodide (PI, BD) were added to discriminate dead cells. Finally, these cells were filtered through a µm cell strainer (BD) to obtain single-suspension cells. Cell dualwavelength analysis and purification were performed on a dual-laser FACS Vantage SE (BD). Hoechst 33342 can be excited at 355 nm UV light and emitted blue fluorescence with a 450/20 band-pass'(BP) filter and red fluorescence with a 675 nm edge filter long-pass (EFLP). A 610 nm dichroic mirror short-pass (DMSP) was used to separate the emission wavelengths. PI-positive (dead) cells were excluded from the analysis.

### VI. Aldefluor assay

High aldehyde dehydrogenase (ALDH) enzyme activity was used to detect lung cancer stem cell populations. The Aldefluor assay was performed according to the manufacturer's guidelines (StemCell Technologies). Briefly, single cells obtained from cell cultures were incubated in an Aldefluor assay buffer containing an ALDH substrate (bodipyaminoacetaldehyde, BAAA) for 50 minutes at 37 "C. As a negative control, a fraction of cells from each sample was incubated under identical conditions in the presence of an ALDH inhibitor (diethylaminobenzaldehyde, DEAB). Flow cytometry was used to measure the ALDHpositive cell population.

### VII. In vivo examination of anti-lung cancer effects mediated by prochlorperazine.

In the first trial, human lung cancer cell line NCI-H441 (purchased from ATCC, 1 million cells/injection) cells were subcutaneously injected into the right flank of NODISCID mice (female, 4-6 weeks old). When tumors became palpable, their sizes were recorded using caliper and mice were randomly divided into control group (DMSO vehicle) and prochlorperazine treatment group (5 mg/kg, 5 days/week, i.p injection). Over the period of 4 weeks, tumorigenesis in both groups was measured using a caliper on a weekly basis. The change in tumor size was expressed as in fold change and plotted over time. Prochlorperazine treatment appeared to suppress and/or delay the growth of tumor as compared to the vehicle control (**p<O.OI).

In the first drug combination test, NCI-H441 cells expressing firefly luciferase (6x105 cells/injection) were injected into NOD/SCID mice (4-6 week of age) via the lateral tail vein for tumor establishment. One week post tumor injection, mice were randomly divided into different groups: vehicle, prochlorperazine (1 mg/kg) in combination with pemetrexed (1 mg/kg) (a standard chemotherapeutic agent for NSCLC), pemetrexed (1 mg/kg) + cisplatin (1 mg/kg) and pemetrexed (1 mg/kg) + prochlorperazine (1 mg/kg) treatment groups. Tumor burden from different groups were recorded by caliper. The change in tumor size was expressed as in fold change and plotted over time. Subsequently, we performed another set of drug combination test. We used two major standard chemotherapeutic regimens in combination with prochlorperazine. For lung adenocarcinoma, the standard drug combination is pemetrexed and cisplatin whereas are used gemcitabine and cisplatin for lung squamous carcinoma. In CL97 (adenocarcinoma) tumor-bearing mice, the combination of pemetrexed + cisplatin+ prochlorperazine provided the most significant tumor suppressive effect than pemetrexed + cisplatin and prochlorperazine alone groups. In CL152 (squamous) tumor-bearing mice, again standard regimen (gemcitabine + cisplatin) added with prochlorperazine appeared to suppress tumorigenesis better than prochlorperazine alone and gemcitabine + cisplatin groups. Next, we demonstrated that prochlorperazine sensitized gefitinib-resistant CL97 lung cancer cells in vivo. CL97 cancer cells (1.5×l06 cells per injection) were injected subcutaneously into NOD/SCID mice to establish in vivo tumor model. Mice were randomly divided into 4 groups: Control, gefitinib alone (100mg/kg, PO, 5 times/week), prochlorperazine alone (5mg/kg, IP, 5 times/week) and gefitinib + prochlorperazine groups. Significant tumor suppressive effect exerted by prochlorperazine alone and gefitinib + prochlorperazine groups were observed 5-week post tumor injection. By week 6 post tumor injection, gefitinib +prochlorperazine group showed the most significant tumor suppressive effect followed by prochlorperazine alone group while both control and gefitinib alone groups demonstrated a similar tumor burden.

### Results

### Cytotoxicity of prochlorperazine in NSCLC cell lines

We first investigated the cytotoxic effect of prochlorperazine in various NSCLC cell lines. A549, A549-0N, CL97, CL141, HCC827, and H441 were lung adenocarcinoma cell lines. CL152, H2l70, and H226 were squamous cell carcinoma cell lines. Cell viability was determined by SRB assay. After treatment for 48 hours with prochlorperazine with various concentrations, the IC50 values in all cancer cell lines were around 20 11M (see Table 1). The experiments were performed to determine the anti-tumor activities of prochlorperazine using clonogenic assay. Clonogenicity of NSCLC cell lines was reduced in a concentration-dependent manner after exposure to different concentration of prochlorperazine. Cancer stem cell (CSC) postulates the existence of a tumor cell population uniquely endowed with self-renewal capacity and therapy resistance. Since prochlorperazine was predicted to preferentially inhibit the lung CSC, we next examined whether the proportion of NSCLC (a lung squamous cell carcinoma cell with wild-type EGFR) spheres could be diminished by prochlorperazine treatment. To determine whether side population (SP) cells, which represent a cell population with CSC characteristics, exist in NSCLC cell lines, we stained the cells with the fluorescent dye, Hoechst 33342, and analyzed the cells with flow cytometry. After the exclusion of dead cells and cellular debris based on the scatter signals, the NSCLC cells contained a small population of cells with SP cell characteristics. After 48 hours of incubation with prochlorperazine at 2.5, 5 and 10 11M, the proportion of SP cells were dosedependently decease (see Table 1).

### Prochlorperazine reduces the proportion of side population cells and ALDIF cells

It was also investigated whether prochlorperazine treatment could deplete the percentage of the cells with ALDH expression (ALDH is an established marker for both hematopoietic and NSCLC CSCs). As shown in Table 1, prochlorperazine treatment also decreased the ALDH+ CL152 population in a dose-dependent manner. In conclusion, prochlorperazine showed low or minimal cytotoxic effects in NSCLC cells.

**Table 1**

| **Cytotoxcities of Prochlorperazine in Various Types of Cancers** | | | | | | | |
|---|---|---|---|---|---|---|---|
| **Cancer type** | **Cell line** | **EGFR nutation status** | **IC50 for prochlmperazine (µM)** | **Clonogenic assay (µM)** | **Sphere formation (µM)** | **Side population (µM)** | **ALDH⁺ assay (µM)** |
| Adenocarcinoma | A549 | WT | 21.5 ± 1.1 | 5 - 10 | | | < 5 |
| | A549-ON (overexpressing Oct4 and Nanog) | WT | 21 ± 1.4 | | | | |
| | CL97 | G719A1 T790M | 13 ± 1.04 | 5 - 10 | 0 - 2.5 | < 5 | < 5 |
| | CL141 | wr | 12.7 ± 1 | 5 - 10 | 0 - 2.5 | > 10 | < 5 |
| | HCC827 | Exon 19 deletion | > 20 | | 5 - 10 | | |
| | H441 | WT | 17.7 ± 1.3 | | 0 - 2.5 | < 5 | < 10 |
| Squamous cell carcinoma | **CL152** | WT | 16.6 ± 2.4 | 5 - 10 | 0 - 5 | < 5 | < 5 |
| | H2170 | WI | 24.7 ± 0.4 | | | | |
| | H226 | wr | 21.2 ± 1.1 | | 0 - 5 | | |
| Large cell carcinoma | H1299 | wr | 27.6 ± 8.8 | 5 - 10 | 0 - 5 | | |
| Liver cancer | Mahlavu | | > 10 | | > 5 | | |
| | SK-Hep1 | | > 20 | | > 5 | | |
| | HepG2 | | > 10 | | | | |
| Colorectal adenocarcinoma | HT29 | | 13.5 ± 0.05 | | 0 - 2.5 | | |
| Brain cancer | GBM8401 | | ~ 10 | 3.33 - 10 | | > 10 | |
| | U87MG | | | | 10 - 15 | > 10 | |
| Breast cancer | MDA-MB-231 | | | | 5 - 10 | | |
| Pancreatic cancer | PANC-1 | | | | 10 - 20 | | |
| Myeloma | J5 | | | | > 5 | | |

In addition, the cytotoxicities of prochlorperazine metabolites at NSCLC parental and sphere cells were examined by SRB assay and Trypan Blue Exclusion method. It was found that N-desmethyl prochlorperazine showed better cytotoxic effect than other tested metabolites in NSCLC cell lines (see Table 2).

**Table 2**

| **IC50 for the Metabolites of Prochlorperazine (not forming part of the invention)** | | | | |
|---|---|---|---|---|
| **IC₅₀ for prochlorperazine metabolites (µM)** | | | | |
| **Compound** | **prochlorperazine** | **N-desmethyl prochlorperazine** | **Prochlorperazine sulfoxide** | **Prochlorperazine sulfoxide 4'-N-oxide** |
| **Structure** | 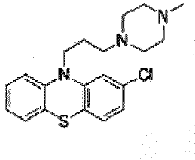 | 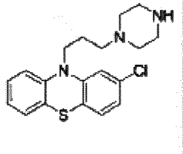 | 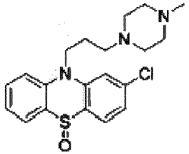 | 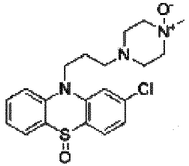 |
| CL141 | 10-20 | 5 - 10 | > 20 | > 20 |
| CL152 | 10-20 | ~ 10 | > 20 | > 20 |
| CL141 spheres | < 5 | < 5 | 5-10 | > 10 |
| CL152 spheres | < 5 | < 5 | > 10 | > 10 |

Also, the effect of the analogs of prochlorperazine on the clonogenicity in CL141 were examined, wherein approximately 1,000 cells were seeded in 6-well plate and treated with different analogs; after 14 days, the colonies were stained with crystal violet, photographed and counted. The results are shown in Table 3, showing that four analogs of prochlorperazine reduced clonogenicity in NSCLC cells.

**Table 3**

| **Clonogenic assay for the Analogs of Prochlorperazine (not forming part of the invention)** | | | | |
|---|---|---|---|---|
| **Clonogenic assay for prochlorperazine analogs (µM)** | | | | |
| **Compound** | **NSC 14948** | **NSC 166184** | **NSC 169470** | **NSC 406619** |
| **Structure** | 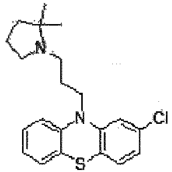 | 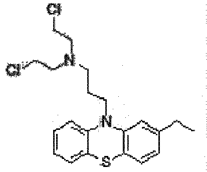 | 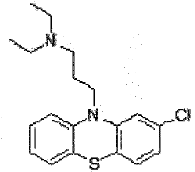 | 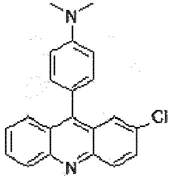 |
| **CL141** | **5-10** | **1-2.5** | **5-10** | **2.5-5** |

### Prochlorperazine induces apoptosis in eL152 spheres and synergistically enhances cytotoxicity in combine with gemcitabine (not part of the invention).

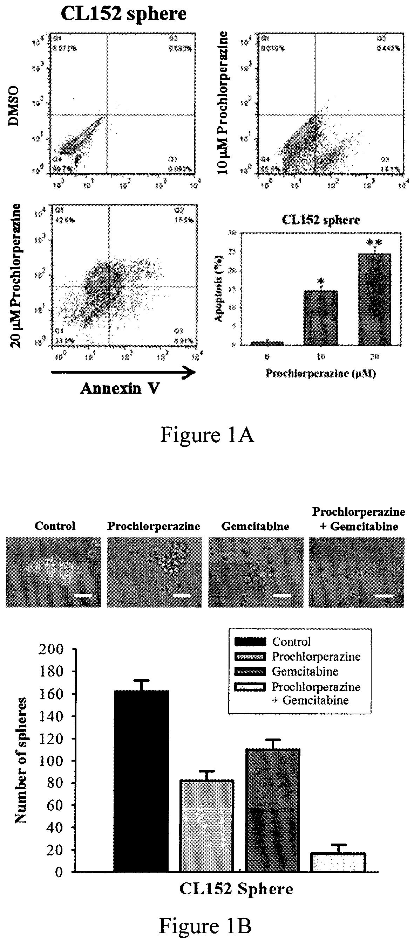

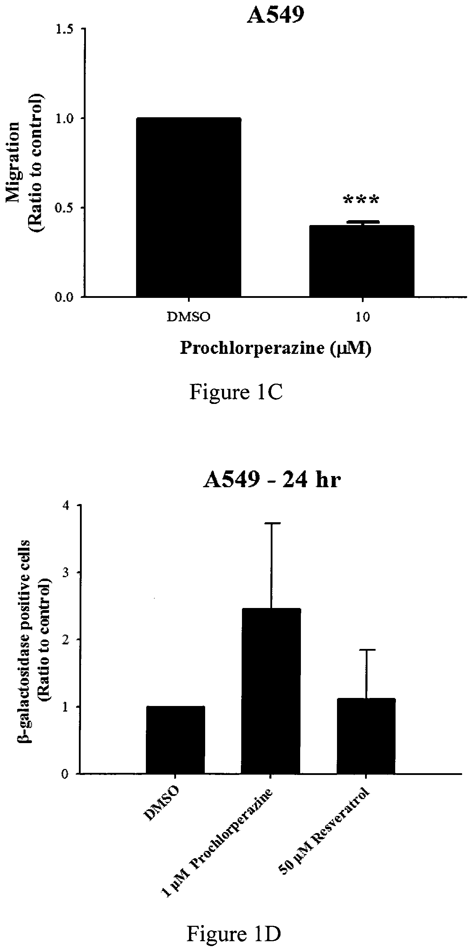

After treatment with prochlorperazine for 48 hours, CL152 spheres were collected and analyzed by flow cytometry. Prochlorperazine led to a dose-dependent increase in apoptosis (Figure 1A). Gemcitabine is a chemotherapeutic agent frequently used in NSCLC treatment. Next, the combination effect of prochlorperazine and gemcitabine was investigated by sphere-formation assay. The result demonstrated that prochlorperazine showed synergistic effect with gemcitabine in CL152 spheres (Figure IB).

### Prochlorperazine inhibits lung cancer cell migration and induces senescence

Cancer cells undergoing epithelial-mesenchymal transition (EMT) have been found to show increased resistance to apoptosis and certain chemotherapeutic drugs. EMT also plays a critical role in the regulation of cancer sternness. EMT is characterized by enhanced cell migration and invasion. Transwell migration assay was used to determine whether subcytotoxic concentration of prochlorperazine could inhibit cell motility of A549 cells in vitro. After 24 hours, treatment with 10 µM prochlorperazine significantly inhibited cell migration of A549 cells as compared with the DMSO controls (Figure 1C). Furthermore, we examined the effect of prochlorperazine on the senescence at A549 cells. β-galactosidase (SA-β-Gal) activity was detected by senescence detection kit (BioVision Inc.). After treatment with 1µ prochlorperazine and 50µ resveratrol as positive control for 24 hours, A549 cells increased β-galactosidase activity and induced β-galactosidase activity positive cells were counted by microscope at 200x field (Figure ID). These data showed that prochlorperazine could induce senescence at NSCLC cells at a low concentration.

### Prochlorperazine significantly inhibits the self-renewal of NSCLC cancer spheres.

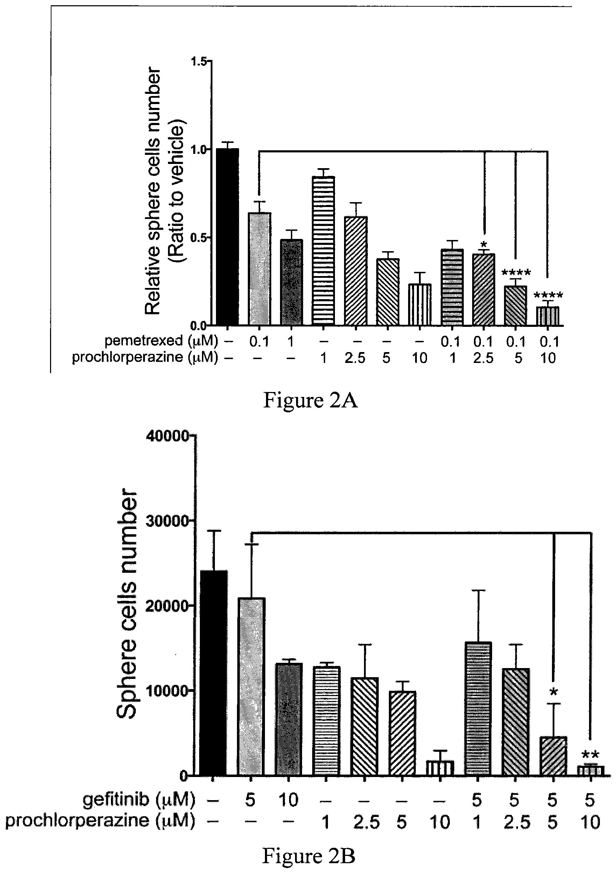

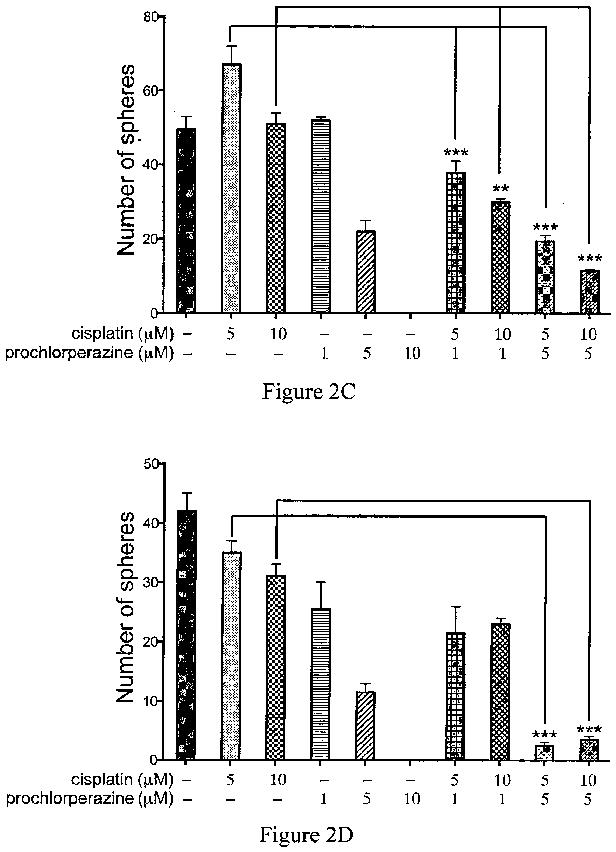

To evaluate whether the prochlorperazine alone or combined with clinical drugs, chemotherapeutic agents and EGFR-tyrosine kinase inhibitors gefitinib, treatment could suppress NSCLC cancer stem-like sphere cells in vitro. CLI41 sphere cells (Figure 2A) and CL97 sphere cells (Figure 2B) were treated with prochlorperazine alone, pemetrexed (CL141 spheres) or gefitinib (CL97 spheres) alone, or a combined treatment for 48 hours. Prochlorperazine (1, 2.5,5 and 10 µM) reduced the number of spheres from the CLI41 and CL97 cells in a concentration-dependent manner (Figure 2A and 2B). The combination, cotreatment with prochlorperazine and clinical drugs, also significantly decreased the number of sphere cells compared to pemetrexed or gefitinib alone (Figure 2A and 2B). To further explore the effect of prochlorperazine combine with cisplatin in CSC, we established the sphere-formation assay of two NSCLC cell lines, including HCC827 and H1299. Prochlorperazine suppressed the sphere-forming ability (Figure 2C and 2D). Interestingly, the combination with cisplatin seemed to have lower spheres number formed by these NSCLC cells than that of cells treated with prochlorperazine or cisplatin alone. Taken together, these data suggest that prochlorperazine has anti-CSC ability on these tested spheres and combination of prochlorperazine with chemotherapeutic agents or EGFR-tyrosine kinase inhibitors may have benefited to cancer therapy.

### In vivo examination of tumor inhibitory effects of prochlorperazine.

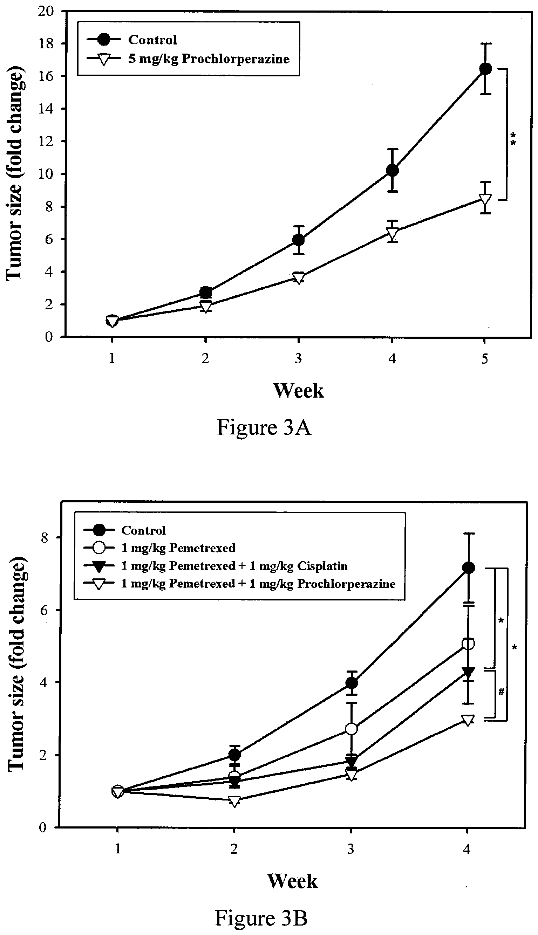

In the first trial (Figure 3A), NCI-H441 (1 × 106 cells/injection) cells were subcutaneously injected into the right flank of NOD/SCID mice (female, 4-6 weeks old). When tumors became palpable, their sizes were recorded using caliper and mice were randomly divided into control group (DMSO vehicle) and prochlorperazine treatment group (5 mg/kg, 5 days/week, i.p, injection). Four weeks post treatment, it was clear that prochlorperazine, at 5 mg/kg, was effective in suppressing tumor growth as compared to the vehicle controls(**p<O.OI).

In the drug combination test (Figure 3B), NCI-H44I cells expressing firefly luciferase (6x105 cells/injection) were injected into NOD/SCID mice (4-6 week of age) via the lateral tail vein for tumor establishment. One week post tumor injection, mice were randomly divided into different groups: control, prochlorperazine (1 mg/kg) in combination with pemetrexed (1 mg/kg) (a standard chemotherapeutic agent for NSCLC), the combination of pemetrexed (1 mg/kg) and cisplatin (1 mg/kg), and the combination of pemetrexed (1 mg/kg) and prochlorperazine (1 mg/kg) groups. Interestingly, we found that using our dosing regimen, the inhibitory effect on tumor growth by cisplatin (at 1 mg/kg) in combination with pemetrexed was not significantly different from that in the pemetrexed alone (1 mg/kg) group (see Figure 3B). However, the combination of prochlorperazine and pemetrexed provided a significantly better tumor inhibitory effect than any other groups (*p<0.05).

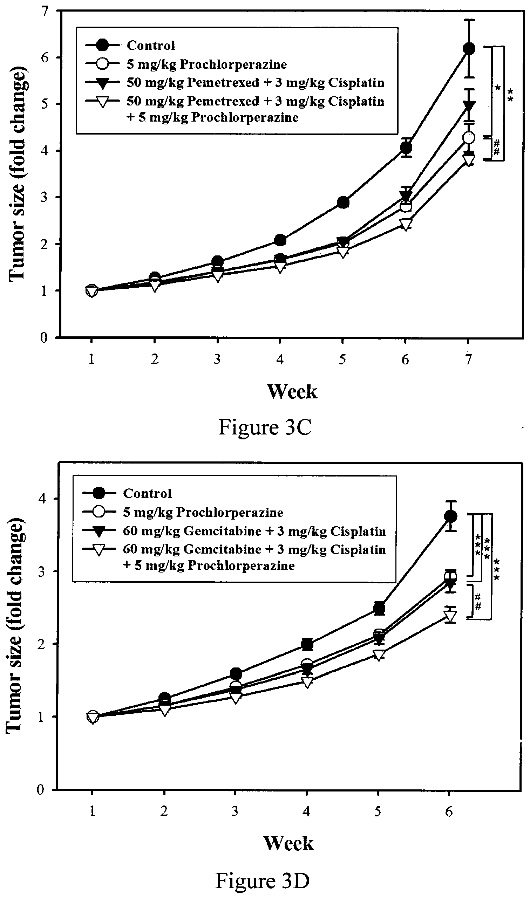

In other drug combination tests, CL97 (Figure 3C) and CL 152 (Figure 3D) cells were subcutaneously injected into the right flank ofNOD/SCID mice (female, 4-6 weeks old). One week post tumor injection, mice were randomly divided into different groups: control group (DMSO vehicle) and prochlorperazine treatment group (5 mg/kg), standard treatment group (50 mg/kg pemetrexed or 60 mg/kg gemcitabine combined with 3 mg/kg cisplatin), and the I:" combination group (standard treatment combined with 5 mg/kg prochlorperazine). From the animal data, prochlorperazine alone could inhibit tumor growth (*p<0.05, ***p<O.OOI); standard treatment combined with prochlorperazine showed more significantly inhibitory effect of tumor growth than other treatments (**p<O.OI, ***p<O.OOI).

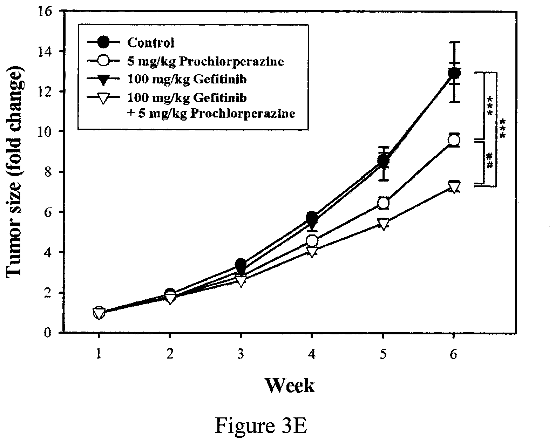

To examine whether prochlorperazine could overcome the drug resistance, CL97 (EGFR T790M and G719A mutations) cells were subcutaneously injected into the right flank of NOD/SCID mice (female, 4-6 weeks old). One week post tumor injection, mice were randomly divided into different groups: control group (DMSO) vehicle) and prochlorperazine treatment group (5 mg/kg), target-therapy group (100 mg/kg gefitinib), and combination group (100 mg/kg gefitinib combined with 5 mg/kg prochlorperazine). In Figure 3E, gefitinib alone was ineffective to inhibit tumor growth. However, prochlorperazine alone or combined with gefitinib showed significantly inhibitory effects of tumor growth (***p<O.OOI). From the evidence, prochlorperazine could overcome the drug resistance through sensitizing chemotherapy or target therapy. This finding suggests that prochlorperazine could be considered as a clinical adjuvant therapeutic agent with chemotherapy or target therapy in the future.

### Clinical cases after prochlorperazine treatment

To prove the concept of the invention, clinical observation study of prochlorperazine (label as drug P in the figures) was performed (see Figures 4-6). The patients include lung squamous cell carcinoma patient treated with Tarceva^{™}(containing erlotinib as active ingredient) (as shown in Figure 4), lung adenocarcinoma patient treated with pemetrexed (as shown in Figure 5), and signet ring cell carcinoma patient treated with a combination of several kinds of chemotherapy, including cisplatin, 5-fluorouracil, irinotecan, paclitaxel, gemcitabine, avastin, TS-1, oxaliplatin, paclitaxel/5-fluorouracil and avastin/gemcitabine/TS-leas shown in Figure 6), who also took prochlorperazine. It was observed that the patients were in a stable or tumor reduction condition. The observations suggested that the addition of prochlorperazine in the current treatment has the benefits to the patients. In other cases, several patients took prochlorperazine for more than 1 year, suggesting the current dose could be tolerated with minimal side effect. Moreover, the addition of prochlorperazine could be considered as a maintenance therapy that helps the patients to control the disease without cancer progression, allowing the patients to live longer. In view of the data including median objective response rate (ORR), progression-free survival (PFS), and overall survival (OS) after prochlorperazine treatment, the disease status of the patients was effectively kept stable and the prolonged survivals of the patients were observed (Figures 7A and 7B).

It can be concluded that prochlorperazine in combination with either gefitinib or erlotinib provides treatment benefits to patients with various cancers, particularly to overcome the drug resistant properties and to prevent cancer metastasis.

It is believed that a person of ordinary knowledge in the art where the present invention belongs can utilize the present invention to its broadest scope based on the descriptions herein with no need of further illustration.

## Claims

1. A pharmaceutical composition for use in treating a gefitinib resistant cancer, which comprises prochlorperazine or a pharmaceutically acceptable salt thereof in combination with gefitinib, wherein the cancer is lung cancer.

2. The pharmaceutical composition for use according to claim 1, wherein the lung cancer is non-small cell lung carcinoma (NSCLC).

3. A pharmaceutical composition for use in preventing cancer metastasis, which comprises prochlorperazine or a pharmaceutically acceptable salt thereof in combination with erlotinib, wherein the cancer is lung cancer.

4. The pharmaceutical composition for use in preventing cancer metastasis of claim 3, wherein the lung cancer is lung squamous cell carcinoma.

## Patentansprüche

1. Pharmazeutische Zusammensetzung zur Verwendung bei der Behandlung eines Gefitinib-resistenten Krebses, welche Prochlorperazin oder ein pharmazeutisch annehmbares Salz davon in Kombination mit Gefitinib umfasst, worin der Krebs Lungenkrebs ist.

2. Pharmazeutische Zusammensetzung zur Verwendung nach Anspruch 1, worin der Lungenkrebs ein nicht-kleinzelliges Lungenkarzinom (NSCLC) ist.

3. Pharmazeutische Zusammensetzung zur Verwendung bei der Verhinderung von Krebsmetastasierung, die Prochlorperazin oder ein pharmazeutisch annehmbares Salz davon in Kombination mit Erlotinib umfasst, worin der Krebs Lungenkrebs ist.

4. Pharmazeutische Zusammensetzung zur Verwendung bei der Verhinderung von Krebsmetastasierung nach Anspruch 3, worin der Lungenkrebs ein Plattenepithelkarzinom der Lunge ist.

## Revendications

1. Composition pharmaceutique à utiliser dans le traitement d'un cancer résistant au géfitinib, comprenant de la prochlorpérazine ou un de ses sels pharmaceutiquement acceptables en association avec le géfitinib, le cancer étant un cancer du poumon.

2. Composition pharmaceutique à utiliser selon la revendication 1, dans laquelle le cancer du poumon est un carcinome pulmonaire non à petites cellules (CPNPC).

3. Composition pharmaceutique à utiliser dans la prévention des métastases cancéreuses, comprenant de la prochlorpérazine ou un de ses sels pharmaceutiquement acceptables en association avec l'erlotinib, dans laquelle le cancer est un cancer du poumon.

4. Composition pharmaceutique à utiliser dans la prévention des métastases cancéreuses de la revendication 3, dans laquelle le cancer du poumon est un carcinome épidermoïde du poumon.
